# EUROPEAN PATENT APPLICATION

(11) **EP 4 806 443 A1**
(43) Date of publication of application: **16.09.2026**
(21) Application number: 24906914.7
(22) Date of filing: 23.10.2024
(51) Int. Cl.: A61K 33/00, A61K 31/337, A61K 31/475, A61K 31/496, A61K 31/5025, A61K 31/506, A61K 31/513, A61K 31/706, A61K 33/24, A61K 45/00, A61P 35/00, A61P 43/00

(54) **ANTICANCER AGENT KIT**

(30) Priority: 22.12.2023 JP 2023216876
(71) Applicant: Nihon Trim Co., Ltd., Osaka 531-0076 (JP)
(72) Inventor: HARA Taichi, Tokyo 169-8050 (JP); KABAYAMA Shigeru, Osaka-shi, Osaka 531-0076 (JP)
(74) Representative: Lorenz Seidler Gossel Part. mbB
(86) International application number: PCT/JP2024/037809
(87) International publication number: WO 2025/134526

(57) **Abstract**

An anticancer agent kit includes: an anticancer activity enhancer having autophagy inhibitory activity and containing molecular hydrogen as an anticancer activity enhancing substance; and an anticancer agent. Targeted cancer cells are at least one of human cervical cancer-derived HeLa cells, human colon cancer-derived Caco-2 cells, or human colorectal cancer-derived HCT116 cells, autophagy of which is inhibited by the molecular hydrogen. The anticancer activity enhancer is electrolyzed hydrogen water, the anticancer agent contains an active ingredient having an autophagy activating effect, and the molecular hydrogen enhances an anticancer effect of the anticancer agent by inhibiting the autophagy activating effect of the anticancer agent.

## Description

### TECHNICAL FIELD

The present invention relates to an anticancer agent kit.

### BACKGROUND ART

The present applicant has made various studies on new applications and functions of various types of hydrogen water such as electrolyzed hydrogen water and mixtures containing the same. For example, Patent Document 1 proposes a hydrogen water mixture for suppressing alcoholic liver disease, in which an ethanol solvent and hydrogen water are mixed with an ethanol concentration of 1% to 4% and a dissolved hydrogen concentration of 550 ppb to 5,600 ppb. The hydrogen water mixture is highly safe, easy and inexpensive to prepare, and can effectively suppress the alcoholic liver disease.

### CITATION LIST

### PATENT DOCUMENT

Patent Document 1: Japanese Unexamined Patent Publication No. 2022-126128

### SUMMARY OF THE INVENTION

### TECHNICAL PROBLEM

A drug therapy (chemotherapy) using an anticancer agent that exhibits anticancer effects by having an effect on the DNA of cancer cells to inhibit the growth of the cancer cells has been used as a method for treating cancer.

Some anticancer agents have strong side effects such as nausea, vomiting, and alopecia, although they have excellent anticancer effects, and increased dose of the anticancer agents increases an economic burden on patients, making it difficult for some patients to continuously use the anticancer agents. There is a demand for reducing the dose of the anticancer agents as much as possible to minimize the side effects and the economic burden on the patients.

The present invention has been made in view of the above circumstances, and an object of the present invention is to provide an anticancer agent kit capable of enhancing the anticancer effect of the anticancer agent without increasing the dose of the anticancer agent.

### SOLUTION TO THE PROBLEM

As a result of intensive studies to achieve the object, the present inventors have found that electrolyzed hydrogen water (dissolved molecular hydrogen) has activity for inhibiting autophagy and that this activity enhances the anticancer effect of the anticancer agent, and have completed the anticancer agent kit of the present invention.

The anticancer agent kit of the present invention includes: an anticancer activity enhancer having autophagy inhibitory activity and containing molecular hydrogen as an anticancer activity enhancing substance; and an anticancer agent.
- A concentration of the molecular hydrogen relative to a reference active ingredient concentration (1 µM) of the anticancer agent is 105 ppb or more and 2,160 ppb or less.
- Targeted cancer cells are at least one selected from the group consisting of human cervical cancer-derived HeLa cells, human colon cancer-derived Caco-2 cells, and human colorectal cancer-derived HCT116 cells, autophagy of which is inhibited by the molecular hydrogen.
- The anticancer activity enhancer is electrolyzed hydrogen water.
- The anticancer agent contains, as an active ingredient, at least one selected from the group consisting of doxorubicin, bosutinib, ponatinib, cisplatin, vincristine, paclitaxel, dasatinib, bosutinib, nilotinib, and fluorouracil, each of which has an autophagy activating effect.
- The molecular hydrogen enhances an anticancer effect of the anticancer agent by inhibiting the autophagy activating effect of the anticancer agent. Note that the anticancer agent kit of the present invention may exclude an anticancer agent kit that is targeted at colon cancer cells and includes an anticancer agent containing fluorouracil as the active ingredient.

The anticancer agent kit of the present invention includes: an anticancer activity enhancer having autophagy inhibitory activity and containing molecular hydrogen as an anticancer activity enhancing substance; and an anticancer agent.
- A concentration of the molecular hydrogen relative to a reference active ingredient concentration (1 µM) of the anticancer agent is 105 ppb or more and 2,160 ppb or less.
- Targeted cancer cells are at least one selected from the group consisting of human cervical cancer-derived HeLa cells, human colon cancer-derived Caco-2 cells, and human colorectal cancer-derived HCT116 cells, autophagy of which is inhibited by the molecular hydrogen.
- The anticancer activity enhancer is electrolyzed hydrogen water.
- The anticancer agent contains, as an active ingredient, at least one selected from the group consisting of doxorubicin, bosutinib, ponatinib, cisplatin, vincristine, paclitaxel, dasatinib, bosutinib, and nilotinib, each of which has an autophagy activating effect.
- The molecular hydrogen enhances an anticancer effect of the anticancer agent by inhibiting the autophagy activating effect of the anticancer agent.

### ADVANTAGES OF THE INVENTION

The present invention can provide an anticancer agent kit capable of enhancing an anticancer effect of an anticancer agent without increasing the dose of the anticancer agent.

### BRIEF DESCRIPTION OF THE DRAWINGS

[FIG. 1] FIG. 1 is a diagram showing the expression levels of LC3 protein (LC3-II) in the presence or absence of Bafilomycin A1 (Baf. A1) by a flux assay using Baf. A1 in mouse embryonic fibroblasts (MEFs).
[FIG. 2] FIG. 2 is a diagram showing the difference between the expression level of LC3-II in the presence of Baf. A1 and the expression level of LC3-II in the absence of Baf. A1 in the MEFs.
[FIG. 3] FIG. 3 is a diagram showing the results of an analysis of autophagy activation by electrolyzed hydrogen water in the MEFs by fluorescence microscopy using an mRFP-GFP-LC3 tandem fluorescent probe.
[FIG. 4] FIG. 4 is a diagram showing the results of an analysis of the autophagy activation by the electrolyzed hydrogen water in human cervical cancer cells (HeLa cells) based on the GFP/RFP ratio using a GFP-LC3-RFP-LC3ΔG probe.
[FIG. 5] FIG. 5 is a diagram showing the results of an analysis of autophagy activation by doxorubicin (anticancer agent) in the HeLa cells based on the GFP/RFP ratio using the GFP-LC3-RFP-LC3ΔG probe.
[FIG. 6] FIG. 6 is a diagram showing the results of verification of a cell growth inhibitory effect of doxorubicin on the HeLa cells.
[FIG. 7] FIG. 7 is a diagram showing the results of verification of apoptosis induction by doxorubicin in the HeLa cells.
[FIG. 8] FIG. 8 is a diagram showing the results of verification of a cell growth inhibitory effect of doxorubicin used in combination with the electrolyzed hydrogen water on the HeLa cells (ATG7 WT).
[FIG. 9] FIG. 9 is a diagram showing the results of verification of the cell growth inhibitory effect of doxorubicin used in combination with the electrolyzed hydrogen water on HeLa cells in which ATG7 (autophagy-related gene) was knocked out (ATG7 KO).
[FIG. 10] FIG. 10 is a diagram showing the results of verification of apoptosis induction by doxorubicin used in combination with the electrolyzed hydrogen water in the HeLa cells.
[FIG. 11] FIG. 11 is a diagram showing the results of an analysis of autophagy activation by doxorubicin used in combination with the electrolyzed hydrogen water in the HeLa cells based on the GFP/RFP ratio using a GFP-LC3-RFP-LC3ΔG probe.
[FIG. 12] FIG. 12 is a diagram showing the results of verification of a cell growth inhibitory effect of doxorubicin on human colon cancer cells (Caco-2 cells) in a serum-free medium or a Baf. A1-containing medium.
[FIG. 13] FIG. 13 is a diagram showing the results of verification of a cell growth inhibitory effect of doxorubicin on the Caco-2 cells in a filtered-water medium or an electrolyzed hydrogen water medium.
[FIG. 14] FIG. 14 is a diagram showing the results of verification of a cell growth inhibitory effect of the electrolyzed hydrogen water on the Caco-2 cells.
[FIG. 15] FIG. 15 is a diagram showing the results of verification of a cell growth inhibitory effect of doxorubicin used in combination with the electrolyzed hydrogen water on the Caco-2 cells in a serum-free medium or a Baf. A1-containing medium.
[FIG. 16] FIG. 16 is a diagram showing the results of verification of apoptosis induction by doxorubicin used in combination with the electrolyzed hydrogen water in the Caco-2 cells.
[FIG. 17] FIG. 17 is a diagram showing the results of an analysis of autophagy activation by doxorubicin used in combination with the electrolyzed hydrogen water in the Caco-2 cells based on the GFP/RFP ratio using a GFP-LC3-RFP probe.
[FIG. 18] FIG. 18 is a diagram showing the results of an analysis of autophagy activation of various types of water in the Caco-2 cells based on the GFP/RFP ratio using a GFP-LC3-RFP probe.
[FIG. 19] FIG. 19 is a diagram showing the results of verification of a cell growth inhibitory effect of doxorubicin used in combination with various types of water on the Caco-2 cells.
[FIG. 20] FIG. 20 is a diagram showing the results of verification of a cell growth inhibitory effect of doxorubicin used in combination with produced hydrogen water on the HeLa cells (ATG7 WT).
[FIG. 21] FIG. 21 is a diagram showing the results of verification of a cell growth inhibitory effect of doxorubicin used in combination with the produced hydrogen water on the HeLa cells in which ATG7 was knocked out (ATG7 KO).
[FIG. 22] FIG. 22 is a diagram showing the results of an analysis of autophagy activation by the electrolyzed hydrogen water in human colorectal cancer cells (HCT116 cells) based on the GFP/RFP ratio using a GFP-LC3-RFP probe.
[FIG. 23] FIG. 23 is a diagram showing the results of an analysis of autophagy activation of paclitaxel (anticancer agent) in the HeLa cells based on the GFP/RFP ratio using a GFP-LC3-RFP-LC3ΔG probe.
[FIG. 24] FIG. 24 is a diagram showing the results of verification of a cell growth inhibitory effect of paclitaxel on the HeLa cells.
[FIG. 25] FIG. 25 is a diagram showing the results of an analysis of autophagy activation by fluorouracil (5-FU, anticancer agent) in the HCT116 cells based on the GFP/RFP ratio using a GFP-LC3-RFP probe.
[FIG. 26] FIG. 26 is a diagram showing the results of verification of a cell growth inhibitory effect of paclitaxel used in combination with the electrolyzed hydrogen water on the HeLa cells (ATG7 WT).
[FIG. 27] FIG. 27 is a diagram showing the results of verification of the cell growth inhibitory effect of paclitaxel used in combination with the electrolyzed hydrogen water on the HeLa cells in which ATG7 (autophagy-related gene) was knocked out (ATG7 KO).
[FIG. 28] FIG. 28 is a diagram showing the results of verification of a cell growth inhibitory effect of fluorouracil used in combination with the electrolyzed hydrogen water on the HCT116 cells.

### DESCRIPTION OF EMBODIMENTS

The anticancer agent kit of the present embodiment includes an anticancer activity enhancer and an anticancer agent. The anticancer agent kit can be targeted at any type of cancer without particular limitations, and is applicable to, for example, cervical cancer, colon cancer, and colorectal cancer.

### <Anticancer Activity Enhancer>

The anticancer activity enhancer means a substance that enhances the effect of an anticancer agent (anticancer effect) by enhancing an action pathway itself of the anticancer agent by the activity of an anticancer activity enhancing substance or by a synergistic action through a pathway different from the action pathway of the anticancer agent. The anticancer activity enhancer of the present embodiment contains an anticancer activity enhancing substance having activity for inhibiting autophagy (hereinafter referred to as "autophagy inhibitory activity") based on the analysis and verification results of the Examples described later.

Autophagy is an intracellular recycling system (a lysosome-dependent degradation system) that degrades intracellular components to purify the intracellular components and remove harmful substances, and acts to prevent various diseases such as carcinogenesis. It is also known that autophagy not only functions as a mechanism for degrading the intracellular components, but also plays a role in regulating biological mechanisms by the secretion of the intracellular components. In particular, it has been found that secretory autophagy (exophagy, extracellular release of secretions) is involved in inflammation regulation and metabolite exchange which are associated with various diseases.

The anticancer activity enhancer of the present embodiment contains molecular hydrogen as the anticancer activity enhancing substance having the autophagy inhibitory activity. In other words, molecular hydrogen can be said to be an active molecule having the autophagy inhibitory activity. The anticancer activity enhancer has an excellent cell growth inhibitory effect on the cancer cell growth by inhibiting autophagy with molecular hydrogen.

The anticancer activity enhancer containing molecular hydrogen may be in any form, such as a liquid containing molecular hydrogen (may be hereinafter referred to as "hydrogen-containing water"), and a gas containing molecular hydrogen (may be hereinafter referred to as "hydrogen-containing gas").

The hydrogen-containing water is a liquid for biological application with dissolved molecular hydrogen. Examples of the liquid for biological application includes water (such as filtered water, purified water, and sterilized water), physiological saline, buffer solutions (such as a phosphate buffer solution, preferably those used for the anticancer agent), infusions (such as a drip and a Ringer's solution that may contain a therapeutic drug), injections, and beverages (e.g., tea beverages such as green tea and black tea, green juice, vegetable juice, and fruit juice). Specific examples of the hydrogen-containing water include electrolyzed hydrogen water produced by electrolysis of water and produced hydrogen water obtained by other methods.

The hydrogen-containing gas may contain at least molecular hydrogen, and may contain other gases (e.g., oxygen, nitrogen, and inert gas) insofar as the object of the invention is not impaired. Specific examples of the hydrogen-containing gas include air containing molecular hydrogen and a mixed gas containing molecular hydrogen and other gas.

The hydrogen-containing water and the hydrogen-containing gas can be produced using a generally commercially available hydrogen generator (e.g., an electrolyzed water generator).

Among the anticancer activity enhancers, the hydrogen-containing water (electrolyzed hydrogen water and produced hydrogen water) is preferable, and the electrolyzed hydrogen water is more preferable because it is highly safe, and easy and inexpensive to prepare with a commercially available device.

The lower limit of the concentration of molecular hydrogen in the anticancer agent kit is preferably 550 ppb or more, more preferably 640 ppb or more, still more preferably 688 ppb or more, much more preferably 896 ppb or more, and even more preferably 1,056 ppb or more. The upper limit of the concentration of molecular hydrogen is preferably 5,600 ppb or less, more preferably 4,000 ppb or less, still more preferably 2,000 ppb or less, much more preferably 1,320 ppb or less, and even more preferably 1,080 ppb or less. The concentration of molecular hydrogen may range, for example, from 550 ppb or more to 5,600 ppb or less, from 800 ppb or more to 1,320 ppb or less, from 640 ppb or more to 1,056 ppb or less, and from 1,056 ppb or more to 1,080 ppb or less. The concentration of molecular hydrogen means the concentration of dissolved hydrogen when the anticancer activity enhancer is the hydrogen-containing water (e.g., electrolyzed hydrogen water).

The concentration of molecular hydrogen relative to a reference active ingredient concentration (1 µM) of the anticancer agent is preferably 105 ppb or more and 2,160 ppb or less.

### <Anticancer Agent>

Although not particularly limited, the anticancer agent preferably contains an active ingredient having the effect of activating autophagy (hereinafter referred to as "autophagy activating effect") as the function corresponding to the autophagy inhibitory activity of the anticancer activity enhancer used in combination with the anticancer agent to prepare the anticancer agent kit. When the anticancer activity enhancer having the autophagy inhibitory activity and the anticancer agent having the autophagy activating effect are co-administered as the anticancer agent kit, the anticancer activity enhancer effectively enhances the anticancer effect by inhibiting the autophagy activating effect of the anticancer agent. This can reduce the dose of the anticancer agent, and can reduce the side effects and the economic burden on the patients.

Examples of the anticancer agent having the autophagy activating effect include the anticancer agents containing, as the active ingredient, doxorubicin, bosutinib, ponatinib, cisplatin, vincristine, paclitaxel, dasatinib, bosutinib, nilotinib, and fluorouracil. The active ingredient of the anticancer agent may be at least one selected from the group consisting of doxorubicin, bosutinib, ponatinib, cisplatin, vincristine, paclitaxel, dasatinib, bosutinib, nilotinib, and fluorouracil. That is, the anticancer agent may contain each of the above active ingredients alone, or may contain two or more of them.

Some anticancer agents having the autophagy activating effect are described in, for example, the following papers. Note that the following papers do not mention anything related to hydrogen.
1) Ponatinib: Diana Corallo, et al. Autophagic flux inhibition enhances cytotoxicity of the receptor tyrosine kinase inhibitor ponatinib. J Exp Clin Cancer Res. 2020.
2) Cisplatin: Ji-Fan Lin, et al. Cisplatin induces protective autophagy through activation of BECN1 in human bladder cancer cells. Drug Des Devel Ther. 2017.
3) Vincristine: ZhenZhen Zhan, et al. Autophagy-mediated HMGB1 release antagonizes apoptosis of gastric cancer cells induced by vincristine via transcriptional regulation of Mcl-1. Autophagy. 2012.
4) Paclitaxel: Jian Wen, et al. Autophagy inhibition re-sensitizes pulse stimulation-selected paclitaxel-resistant triple negative breast cancer cells to chemotherapy-induced apoptosis. Breast Cancer Res Treat. 2015.
5) Dasatinib: Xiao-Feng Le, et al. Dasatinib induces autophagic cell death in human ovarian cancer. Cancer. 2010.
6) Nilotinib: Hui-Chuan Yu, et al. Nilotinib induces autophagy in hepatocellular carcinoma through AMPK activation. J Biol Chem. 2013.

As described above, the electrolyzed hydrogen water (dissolved molecular hydrogen) inhibits autophagy. An anticancer agent containing, for example, doxorubicin, as the active ingredient activates autophagy, as can be seen from the results of the analysis and verification in the Examples described later. Based on the hypothesis that autophagy activation might attenuate the anticancer effect of the anticancer agent having the autophagy activating effect, the present inventors had further studies and found the possible use of the physiological action of molecular hydrogen, that is, autophagy inhibition. The anticancer agent kit of the present invention, which has been completed based on the findings, enhances the anticancer effect more than before by effectively inhibiting the autophagy activating effect of the anticancer agent by using the activity of molecular hydrogen.

The concentration of the anticancer agent (the total concentration when two or more active ingredients are contained) in the anticancer agent kit (relative to the total amount of the components of the anticancer agent kit) is not particularly limited, and may be the concentration of the active ingredient applied to the treatment of targeted cancer cells. For example, as described in the Examples below, the concentration of the active ingredient in the anticancer agent is about 0.5 µM to about 2 µM for the human cervical cancer-derived HeLa cells. The concentration of the active ingredient in the anticancer agent is about 1 µM to about 4 µM for human colon cancer-derived Caco-2 cells. The concentration of the active ingredient in the anticancer agent is about 1 µM to about 10 µM for human colorectal cancer-derived HCT116 cells.

### <Anticancer Agent Kit>

The form of the anticancer agent kit is not particularly limited, and may be appropriately determined depending on the form of the anticancer activity enhancer and the anticancer agent. For example, an anticancer agent kit including the hydrogen-containing water (electrolyzed hydrogen water) as the anticancer activity enhancer may be in the form of a liquid containing the anticancer agent dissolved in the hydrogen-containing water. The administration of the liquid anticancer agent kit may include oral administration, injection such as drip infusion, or parenteral administration such as subcutaneous administration and transdermal administration. The administration of the anticancer agent kit including the hydrogen-containing gas as the anticancer activity enhancer may include, for example, pulmonary administration of the hydrogen-containing gas by inhalation or aspiration and oral or parenteral administration of the anticancer agent. Thus, the anticancer activity enhancer and the anticancer agent may be administered by different methods (may be administered separately) depending on their forms. In this case, they may be administered simultaneously or at different times.

From the viewpoint of enhancing the anticancer effect more than before by effectively inhibiting the autophagy activating effect of the anticancer agent with the autophagy inhibitory activity of molecular hydrogen, the cancer cells targeted by the anticancer agent kit are not particularly limited as long as they are cancer cells whose autophagy is inhibited by molecular hydrogen. In other words, the anticancer agent kit can be effectively applied to any of such cancer cells, and the cancer cells are not particularly limited to those described in the Examples.

In the anticancer agent kit, the concentration of the active ingredient in the anticancer agent is preferably 0.5 µM or more and 10 µM or less, and the concentration of molecular hydrogen in the anticancer agent kit (the total amount of the components) is preferably 1,056 ppb or more and 1,080 ppb or less. The concentration of the active ingredient in the anticancer agent is not limited to the above range, and may be appropriately determined according to the concentration of the active ingredient, applied to the treatment of the targeted cancer cells, for example, 0.5 µM or more and 5 µM or less, 0.5 µM or more and 4 µM or less, 0.5 µM or more and 2 µM or less, or 0.5 µM or more and 1 µM or less.

The anticancer agent kit constituted as described above can reduce the dose of the anticancer agent by enhancing the anticancer effect of the anticancer agent, and as a result, can solve the problems such as the side effects and the economic burden on the patients at once.

### [Examples]

The present invention will be described below based on the Examples. The present invention is not limited to these Examples, and these Examples can be modified and changed in accordance with the spirit of the present invention. Such modifications and changes are not excluded from the scope of the present invention. In the following diagrams, * indicates p < 0.05, ** indicates p < 0.01, and *** indicates p < 0.001.

### <1. Production of Electrolyzed Hydrogen Water>

Electrolyzed hydrogen water samples with different dissolved hydrogen concentration levels of 1 to 4 were obtained by using an electrolyzed water generator (manufactured by Nihon Trim Co., Ltd., trade name: TRIM ION GRACE) under the conditions of a temperature of 22°C and a flow rate of 1.5 L/min, and filtered water was obtained by filtration with micro carbon.

Next, the pH and dissolved hydrogen concentration of the electrolyzed hydrogen water of each level were measured. A pH meter (manufactured by HORIBA, Ltd., trade name: LAQUAact D-71) was used for the measurement of pH, and a dissolved hydrogen meter DH-35A (manufactured by DKK-TOA CORPORATION) was used for the measurement of the dissolved hydrogen concentration.
- Level 1: 800 ppb, pH 7
- Level 2: 860 ppb, pH 8
- Level 3: 1,120 ppb, pH 9
- Level 4: 1,320 ppb (to 1,350 ppb), pH 10

### <Preparation of Medium for Treating Electrolyzed Hydrogen Water (Electrolyzed Hydrogen Water Medium)>

5 × Dulbecco's modified Eagle's media (hereinafter referred to as "DMEMs") each prepared from powder were diluted 5-fold either with the filtered water or the electrolyzed hydrogen water of different levels, and used as media for treating the electrolyzed hydrogen water (i.e., hydrogen water mixtures as treatment media obtained by mixing 20% 5 × DMEM with 80% electrolyzed hydrogen water or filtered water). The dissolved hydrogen concentrations of the electrolyzed hydrogen water medium of different levels were as follows.
- Level 1: 800 ppb × 4/5 = 640 ppb
- Level 2: 860 ppb × 4/5 = 688 ppb
- Level 3: 1,120 ppb × 4/5 = 896 ppb
- Level 4: 1,320 ppb (to 1,350 ppb) × 4/5 = 1,056 ppb (to 1,080 ppb)

### <2. Analysis of Autophagy Inhibition by Electrolyzed Hydrogen Water (Analysis from Multiple Perspectives)>

Whether the electrolyzed hydrogen water inhibits autophagy was analyzed from multiple perspectives.

### (2-1) Flux Assay with Bafilomycin A1

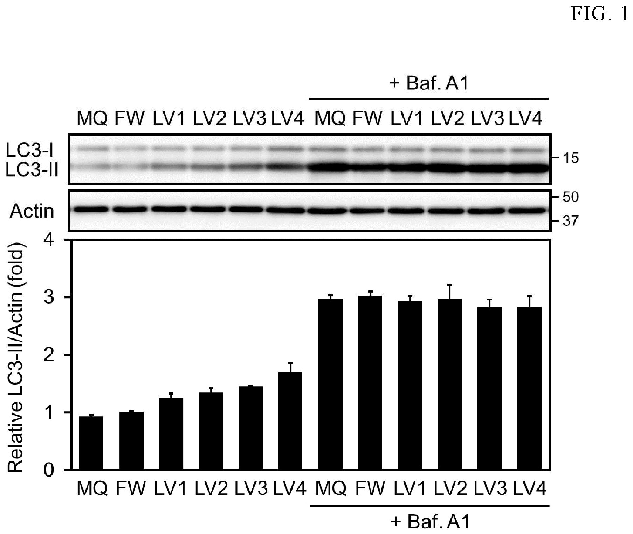

Mouse embryonic fibroblasts (hereinafter referred to as "MEFs") were seeded into a 6-well plate at 5.0 × 10⁵ cells/well and cultured in DMEM under the conditions of 37°C and 5% CO₂ for 24 hours (this process may be hereinafter referred to as "preculture"). After the preculture, the medium was exchanged with various DMEMs shown below in the absence or presence of 200 nM Bafilomycin A1 (an inhibitor, hereinafter referred to as "Baf. A1"), and the cells were cultured for 4 hours. The treated cells were then lysed with a lysis buffer, and western blotting was performed on the lysate to analyze the expression level of LC3 protein (hereinafter referred to as "LC3-II") with a specific antibody against LC3-II. FIG. 1 shows the results.

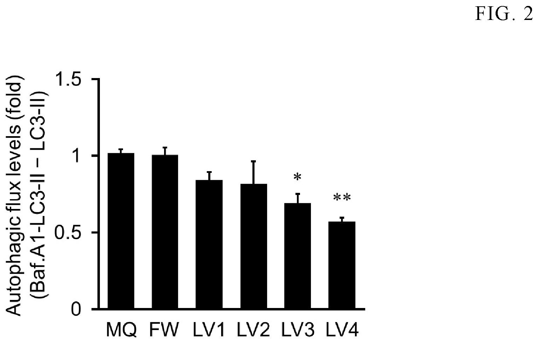

The expression level of LC3-II shown in FIG. 1 correlates with the number of autophagosomes. However, the expression level of LC3-II at a certain time point alone does not indicate the actual flux level, and it is unclear whether autophagy is activated or inhibited. Thus, the expression level of LC3-II transferred to lysosomes was measured by comparing the expression levels of LC3-II in the presence and absence of Baf. A1. Specifically, autophagic flux was analyzed by calculating the difference between the expression level of LC3-II in the presence of Baf. A1 and the expression level of LC3-II in the absence of Baf. A1. FIG. 2 shows the results.

In FIGS. 1 and 2, MQ represents DMEM containing ultrapure water (ultrapure water medium) diluted with ultrapure water alone in the section of <Preparation of Medium for Treating Electrolyzed Hydrogen Water (Electrolyzed Hydrogen Water Medium)>, FW represents DMEM containing filtered water (filtered-water medium) diluted with filtered water alone in the section of <Preparation of Medium for Treating Electrolyzed Hydrogen Water (Electrolyzed Hydrogen Water Medium)>, LV1 to LV4 represent DMEMs containing electrolyzed hydrogen water with dissolved hydrogen concentration levels of 1 to 4 (electrolyzed hydrogen water media), and +Baf. A1 represents media dissolving 200 nM Baf. A1 in the above DMEMs (Baf. A1-containing media).

FIG. 1 confirmed that the expression level of LC3-II was higher in the absence of Baf. A1 in the electrolyzed hydrogen water (LV1 to LV4) than in the ultrapure water (MQ) and the filtered water (FW), and increased in proportion to the dissolved hydrogen concentration. In the presence of Baf. A1 (+Baf. A1), the expression level of LC3-II in the electrolyzed hydrogen water was equivalent to the expression levels in the ultrapure water and the filtered water at any dissolved hydrogen concentration.

FIG. 2 shows that the difference between the expression level of LC3-II in the presence of Baf. A1 and the expression level of LC3-II in the absence of Baf. A1 was smaller in the electrolyzed hydrogen water (LV1 to LV4) than in the ultrapure water (MQ) and the filtered water (FW), and that the difference decreased as the dissolved hydrogen concentration increased. These results indicate that the electrolyzed hydrogen water inhibits the degradation by autolysosomes because LC3-II accumulates more (the amount of degradation by autophagy decreases) as the dissolved hydrogen concentration increases.

### (2-2) Fluorescence Microscopy Using mRFP-GFP-LC3 Tandem Fluorescent Probe

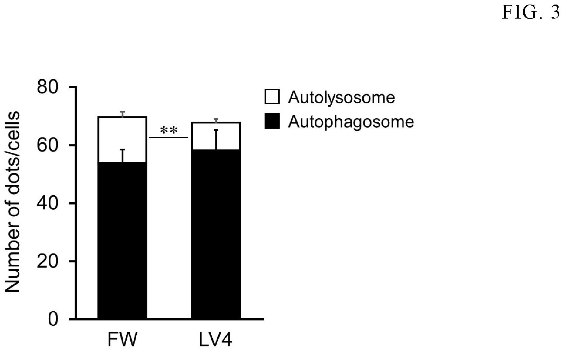

For MEFs expressing an mRFP-GFP-LC3 tandem fluorescent probe (hereinafter referred to as "tf-LC3 probe"), the medium was exchanged with various DMEMs shown below, and the cells were cultured for 4 hours. The MEFs were then fixed with 2% paraformaldehyde. Intracellular fluorescence was observed with a confocal laser scanning microscope, and the number of autolysosomes was measured. FIG. 3 shows the results.

In FIG. 3, FW represents DMEM containing filtered water (filtered-water medium) diluted with filtered water alone in the section of <Preparation of Medium for Treating Electrolyzed Hydrogen Water (Electrolyzed Hydrogen Water Medium)>, and EHW LV4 represents DMEM containing electrolyzed hydrogen water with a dissolved hydrogen concentration level of 4 (electrolyzed hydrogen water medium).

The tf-LC3 probe is a circular DNA (plasmid) having genes producing fluorescent proteins that allow monitoring of autophagy, and is described in, for example, the following paper. The MEFs expressing the tf-LC3 probe refer to cells that produce (express) fluorescent proteins for monitoring autophagy by delivering the plasmid into the cells and incorporating (genetically modifying) the probe genes into the genome of the MEFs. The tf-LC3 probe uses the difference in sensitivity to acidity, and the GFP fluorescence is quenched under acidic conditions (pH < 5.0), whereas the mRFP fluorescence is relatively stable and retained even in lysosomes. In other words, the tf-LC3 probe can visualize the transition from the autophagosomes to acidic autolysosomes.
Shunsuke Kimura, Takeshi Noda, Tamotsu Yoshimori. Dissection of the autophagosome maturation process by a novel reporter protein, tandem fluorescent-tagged LC3. Autophagy. 2007.3(5): 452-60. doi: 10.4161/auto.4451.

FIG. 3 confirmed that the number of autolysosomes decreased in the electrolyzed hydrogen water (EHW LV4). The results indicate that the electrolyzed hydrogen water inhibits the degradation by autolysosomes.

### (2-3) Analysis of Autophagic Activity based on GFP/RFP Ratio with GFP-LC3-RFP-LC3ΔG Probe

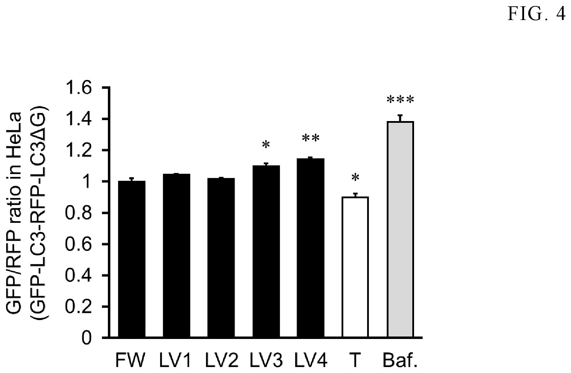

Human cervical cancer cells (hereinafter referred to as "HeLa cells") expressing a GFP-LC3-RFP-LC3ΔG probe were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 4 hours. Then, the cells were detached and suspended, and the fluorescence intensities of GFP and RFP were analyzed by flow cytometry. The autophagic activity was quantitatively analyzed by determining the GFP/RFP ratio. FIG. 4 shows the results.

In FIG. 4, FW represents DMEM containing filtered water (filtered-water medium) diluted with filtered water alone in the section of <Preparation of Medium for Treating Electrolyzed Hydrogen Water (Electrolyzed Hydrogen Water Medium)>, LV1 to LV4 represent DMEMs containing electrolyzed hydrogen water with dissolved hydrogen concentration levels of 1 to 4 (electrolyzed hydrogen water media), T represents a medium (Torin 1-containing filtered-water medium) containing 1 µM Torin 1 dissolved in DMEM containing electrolyzed hydrogen water with a dissolved hydrogen concentration level of 4 (electrolyzed hydrogen water medium), and Baf. represents a medium (Baf. A1-containing filtered-water medium) containing 200 nM Baf. A1 dissolved in DMEM containing electrolyzed hydrogen water with a dissolved hydrogen concentration level of 4 (electrolyzed hydrogen water medium).

The GFP-LC3-RFP-LC3ΔG probe (or a GFP-LC3-RFP probe described later) is described in, for example, the following paper. The GFP-LC3-RFP-LC3ΔG probe (or GFP-LC3-RFP probe) is cleaved by ATG4, and the same number of GFP-LC3 and RFP-LC3ΔG (or RFP) are produced in the cytoplasm. GFP-LC3 is localized in the autophagosomes, and then quenched and degraded in the autolysosomes. RFP-LC3ΔG (or RFP) remains in the cytoplasm and serves as an internal control reflecting the amount of GFP-LC3. The GFP/RFP ratio is obtained by quantifying the cumulative amount of degradation of GFP-LC3 by autophagy. A decrease in the GFP/RFP ratio indicates autophagy activation. An increase in the GFP/RFP ratio indicates autophagy inhibition.
Takeshi Kaizuka, Hideaki Morishita, Yutaro Hama, Satoshi Tsukamoto, Takahide Matsui, Yuichiro Toyota, Akihiko Kodama, Tomoaki Ishihara, Tohru Mizushima, Noboru Mizushima. An Autophagic Flux Probe that Releases an Internal Control. Mol Cell. 2016; 64(4): 835-849. doi: 10.1016/j.molcel.2016.09.037.

FIG. 4 shows that GFP accumulated more as the dissolved hydrogen concentration in the electrolyzed hydrogen water (LV1 to LV4) increased, and the GFP/RFP ratio increased, indicating that the electrolyzed hydrogen water inhibited autophagy in the human cervical cancer-derived HeLa cells.

### (2-4) Conclusion

The results of the analyses from the multiple perspectives (2-1) to (2-3) suggest that the electrolyzed hydrogen water inhibits autophagy, that is, has activity for inhibiting autophagy (autophagy inhibitory activity).

### <3. Analysis of Autophagy Activation by Anticancer Agent Doxorubicin and Verification of Anticancer Effect>

Whether the anticancer agent doxorubicin activated autophagy and whether the autophagy activation influenced the anticancer effect were verified.

### (3-1) Analysis of Autophagic Activity of Doxorubicin based on GFP/RFP Ratio with GFP-LC3-RFP-LC3ΔG Probe

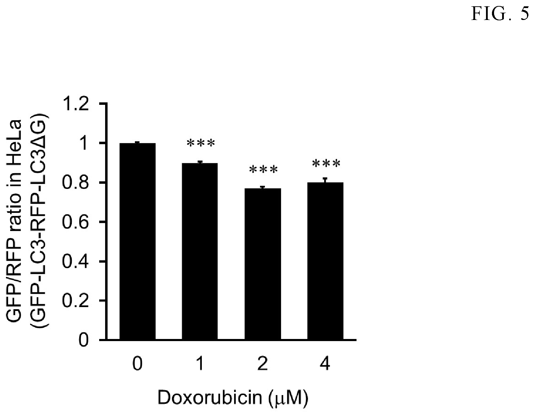

HeLa cells expressing the GFP-LC3-RFP-LC3ΔG probe were seeded into a 12-well plate at 1.0 × 10⁵ cells/well and cultured (precultured) in DMEM under the conditions of 37°C and 5% CO₂ for 24 hours. After the preculture, the medium was exchanged with DMEM supplemented with an anticancer agent containing doxorubicin (Sigma-Aldrich Co., LLC.) at a predetermined concentration (1, 2, or 4 µM), and the cells were cultured for 4 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 5 shows the results.

FIG. 5 shows that the GFP/RFP ratio decreased as the doxorubicin concentration increased, contrary to the electrolyzed hydrogen water (see FIG. 4), indicating that doxorubicin has the effect of activating autophagy (autophagy activating effect). From the results, it is predicted that doxorubicin attenuates the anticancer effect by activating autophagy.

### (3-2) Verification of Cell Growth Inhibitory Effect

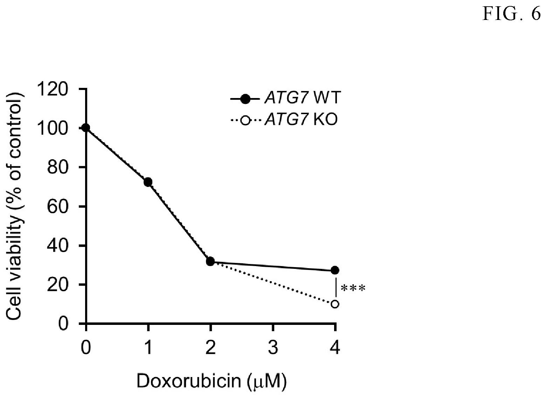

HeLa cells (ATG7 WT) or HeLa cells in which ATG7 (autophagy-related gene) was knocked out (ATG7 KO) were seeded into a 96-well plate at 1.0 × 10⁴ cells/well and cultured (precultured) in DMEM under the conditions of 37°C and 5% CO₂ for 24 hours. After the preculture, the medium was exchanged with DMEM supplemented with an anticancer agent containing doxorubicin at a predetermined concentration (1, 2, or 4 µM), and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIG. 6 shows the results.

### (3-3) Verification of Apoptosis Induction

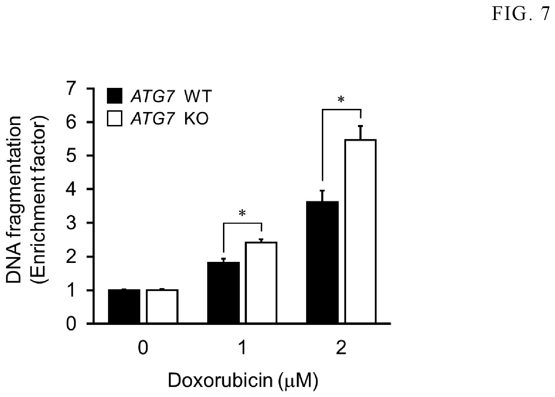

Apoptosis induction was assessed using Cell Death Detection ELISAPLUS (Roche Diagnostics, Basel, Switzerland) according to the manual. HeLa cells (ATG7 WT) or HeLa cells in which ATG7 was knocked out (ATG7 KO) were seeded into a 12-well plate at 1.0 × 10⁵ cells/well and cultured (precultured) in DMEM under the conditions of 37°C and 5% CO₂ for 24 hours. After the preculture, the medium was exchanged with DMEM supplemented with an anticancer agent containing doxorubicin at a predetermined concentration (1 or 2 µM), and the cells were cultured for 24 hours. The cells were then lysed with a lysis buffer, and the lysate was added to each well of the ELISA plate, incubated with an immunological reagent buffer containing anti-histone biotin and anti-DNA POD for 2 hours, and washed with an incubation buffer. Then, the resultant was reacted with 100 µL of an ABTS substrate buffer, and the absorbance was measured at 405 nm and 490 nm. FIG. 7 shows the results.

The comparison between ATG7 WT and ATG7 KO in FIGS. 6 and 7 confirmed that the knockout of ATG7 (autophagy-related gene) enhanced the anticancer effect of doxorubicin (cell growth inhibitory effect and apoptosis induction) in proportion to the concentration.

### (3-4) Conclusion

In consideration of the results in the above sections (3-1) to (3-3), an anticancer agent such as doxorubicin having the autophagy activating effect is expected to enhance the anticancer effect when used in combination with the electrolyzed hydrogen water that inhibits autophagy.

### <4. Verification 1 of Anticancer Effect Enhancement by Combined Use of Electrolyzed Hydrogen Water and Anticancer Agent Doxorubicin>

Whether the combined use of the electrolyzed hydrogen water and the anticancer agent doxorubicin (anticancer agent kit) enhanced the anticancer effect on the human cervical cancer-derived HeLa cells was verified.

### (4-1) Verification of Cell Growth Inhibitory Effect

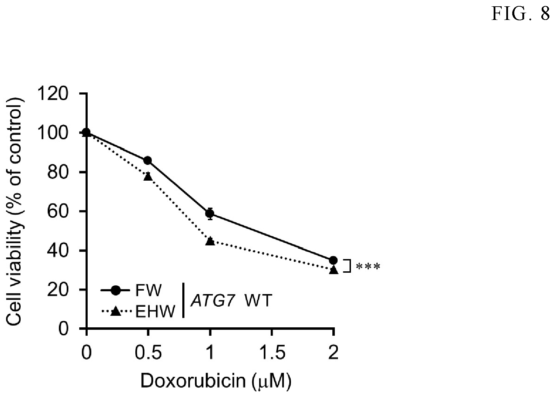

HeLa cells (ATG7 WT) or HeLa cells in which ATG7 (autophagy-related gene) was knocked out (ATG7 KO) were seeded into a 96-well plate at 1.0 × 10⁴ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. The results are shown in FIGS. 8 (ATG7 WT) and 9 (ATG7 KO).

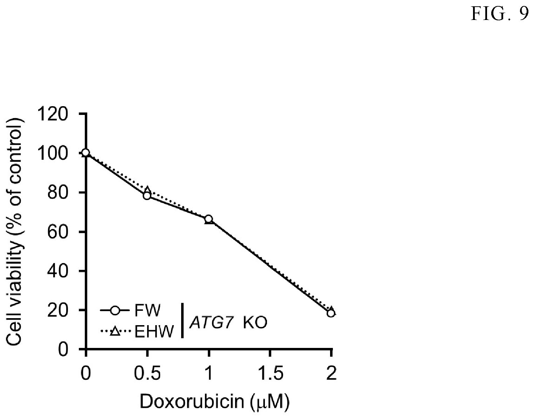

In FIGS. 8 and 9, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which doxorubicin was added at a predetermined concentration (0.5, 1, or 2 µM), and EHW represents DMEM containing electrolyzed hydrogen water with a dissolved hydrogen concentration level of 4 (electrolyzed hydrogen water medium), to which doxorubicin was added at the predetermined concentration.

FIG. 8 (ATG7 WT) confirmed that the combined use of the electrolyzed hydrogen water and doxorubicin enhanced the cell growth inhibitory effect of doxorubicin. FIG. 9 (ATG7 KO) confirmed that the enhancing effect of the combined use was lost due to the knockout of ATG7 (autophagy-related gene).

### (4-2) Verification of Apoptosis Induction

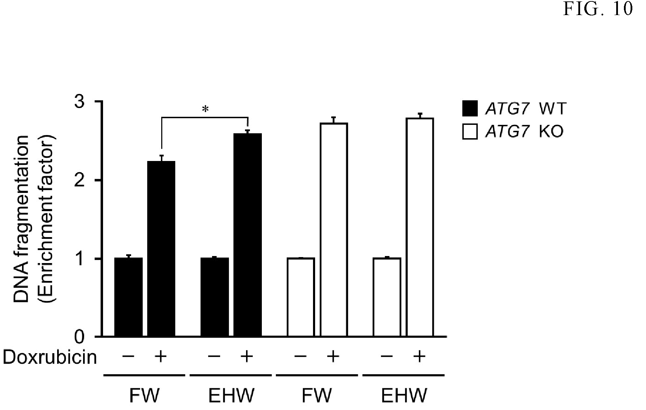

Apoptosis induction was assessed using Cell Death Detection ELISAPLUS (Roche Diagnostics, Basel, Switzerland) according to the manual. HeLa cells (ATG7 WT) or HeLa cells in which ATG7 (autophagy-related gene) was knocked out (ATG7 KO) were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 24 hours. The cells were then lysed with a lysis buffer, and the lysate was added to each well of the ELISA plate, incubated with an immunological reagent buffer containing anti-histone biotin and anti-DNA POD for 2 hours, and washed with an incubation buffer. Then, the resultant was reacted with 100 µL of an ABTS substrate buffer, and the absorbance was measured at 405 nm and 490 nm. FIG. 10 shows the results.

In FIG. 10, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which no doxorubicin was added (-) or 1 µM doxorubicin was added (+), and EHW represents DMEM containing electrolyzed hydrogen water with a dissolved hydrogen concentration level of 4 (electrolyzed hydrogen water medium), to which no doxorubicin was added (-) or 1 µM doxorubicin was added (+).

ATG7 WT shown in FIG. 10 confirmed that the combined use of the electrolyzed hydrogen water and doxorubicin enhanced the apoptosis-inducing effect of doxorubicin. ATG7 KO shown in FIG. 10 confirmed that the enhancing effect of the combined use was lost due to the knockout of ATG7 (autophagy-related gene).

### (4-3) Analysis of Autophagic Activity

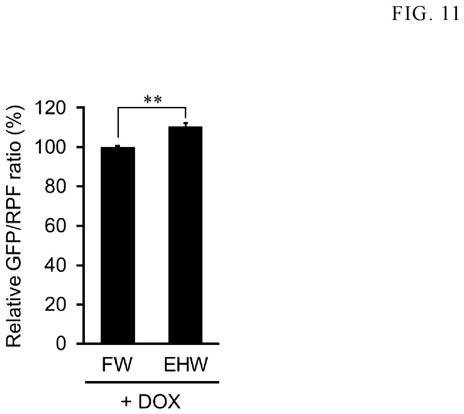

HeLa cells (ATG7 WT) expressing the GFP-LC3-RFP-LC3ΔG probe were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 4 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 11 shows the results.

In FIG. 11, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which 1 µM doxorubicin was added, and EHW represents DMEM containing electrolyzed hydrogen water with a dissolved hydrogen concentration level of 4 (electrolyzed hydrogen water medium), to which 1 µM doxorubicin was added.

As shown in FIG. 11, the combined use of the electrolyzed hydrogen water and doxorubicin increased the GFP/RFP ratio, indicating that the electrolyzed hydrogen water inhibits the autophagy activation by doxorubicin.

### (4-4) Summary

In consideration of the results in the above sections (4-1) to (4-3), the electrolyzed hydrogen water is expected to enhance the anticancer effect by inhibiting the autophagy activating effect of the anticancer agent such as doxorubicin in the human cervical cancer cells (HeLa cells).

### <5. Verification 2 of Anticancer Effect Enhancement by Combined Use of Electrolyzed Hydrogen Water and Anticancer Agent Doxorubicin>

Whether the combined use of the electrolyzed hydrogen water and the anticancer agent doxorubicin (anticancer agent kit) enhanced the anticancer effect on the human colon cancer-derived Caco-2 cells was verified.

### (5-1) Verification of Cell Growth Inhibitory Effect

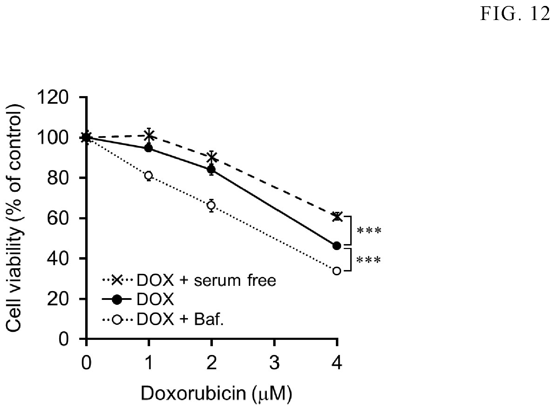

Human colon cancer cells (hereinafter referred to as "Caco-2 cells") were seeded into a 96-well plate at 2.0 × 10⁴ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIGS. 12 to 15 show the results.

In FIG. 12, DOX + serum free represents DMEM with no serum (serum-free medium) to which doxorubicin was added at a predetermined concentration (1, 2, or 4 µM), DOX represents serum-containing DMEM with doxorubicin added at a predetermined concentration, and DOX + Baf. represents a medium (Baf. A1-containing medium) containing 200 nM Bafilomycin A1 (Baf. A1) dissolved in serum-containing DMEM, to which doxorubicin was added at the predetermined concentration.

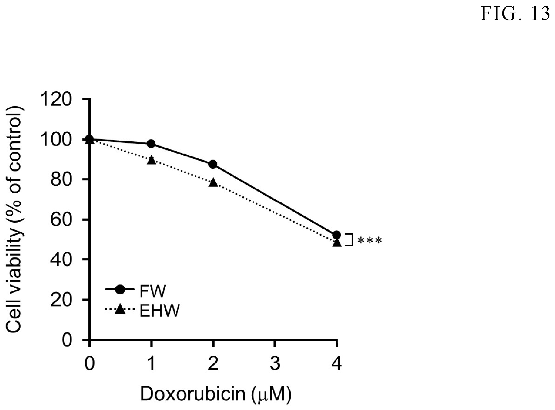

In FIG. 13, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which doxorubicin was added at a predetermined concentration (1, 2, or 4 µM), and EHW represents DMEM containing electrolyzed hydrogen water with a dissolved hydrogen concentration level of 4 (electrolyzed hydrogen water medium), to which doxorubicin was added at the predetermined concentration.

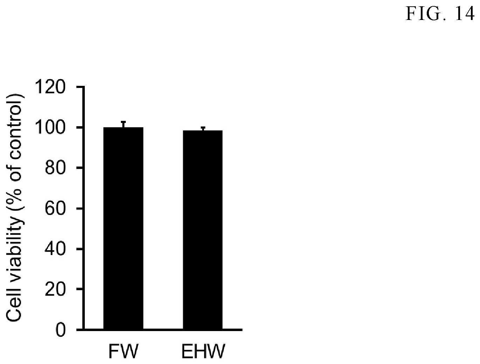

In FIG. 14, FW represents DMEM containing filtered water (filtered-water medium) diluted with filtered water alone in the section of <Preparation of Medium for Treating Electrolyzed Hydrogen Water (Electrolyzed Hydrogen Water Medium)>, and EHW represents DMEM containing electrolyzed hydrogen water with a dissolved hydrogen concentration level of 4 (electrolyzed hydrogen water medium).

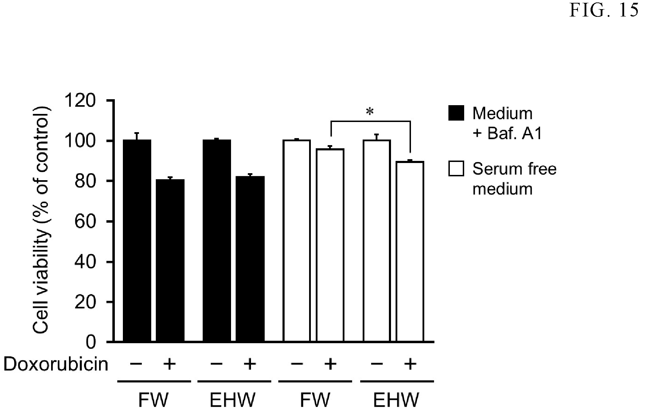

In FIG. 15, FW (Medium + Baf. A1) represents a medium (Baf. A1-containing filtered-water medium) containing 200 nM Baf. A1 dissolved in DMEM containing filtered water and a serum prepared according to the section <Preparation of Medium for Treating Electrolyzed Hydrogen Water (Electrolyzed Hydrogen Water Medium)>, to which no doxorubicin was added (-) or 2 µM doxorubicin was added (+), EHW (Medium + Baf. A1) represents a medium (Baf. A1-containing electrolyzed hydrogen water medium) containing 200 nM Baf. A1 dissolved in DMEM containing electrolyzed hydrogen water with a dissolved hydrogen concentration level of 4 and a serum, to which no doxorubicin was added (-) or 2 µM doxorubicin was added (+) in the same manner, FW (Serum free medium) represents serum-free DMEM containing filtered water (serum-free filtered-water medium) to which no doxorubicin was added (-) or 2 µM doxorubicin was added (+) in the same manner, and EHW (Serum free medium) represents serum-free DMEM containing electrolyzed hydrogen water of a dissolved hydrogen concentration level of 4 (serum-free electrolyzed hydrogen water medium) to which no doxorubicin was added (-) or 2 µM doxorubicin was added (+) in the same manner as the above.

FIG. 12 confirmed that the combined use (DOX + Baf.) of doxorubicin and the autophagy inhibitor (Baf. A1) enhanced the cell growth inhibitory effect of doxorubicin. On the other hand, it was confirmed that the cell growth inhibitory effect of doxorubicin was rather reduced under autophagy activating conditions (DOX + serum free). FIG. 13 confirmed that the combined use of the electrolyzed hydrogen water and doxorubicin (EHW) enhanced the cell growth inhibitory effect. Whether this enhancing effect is achieved by the autophagy inhibitory activity of the electrolyzed hydrogen water will be further verified below.

FIGS. 14 and 15 confirmed that when doxorubicin and the autophagy inhibitor (Baf. A1) were used in combination ("Medium + Baf. A1," under the autophagy inhibiting conditions), there was no significant difference between the filtered water (FW) and the electrolyzed hydrogen water (EHW), indicating that the cell growth inhibitory effect is not changed by the combined use of doxorubicin and the electrolyzed hydrogen water. On the other hand, under the autophagy activating conditions in the serum-free medium without doxorubicin and Baf. A1, the comparison between the filtered water (FW) and the electrolyzed hydrogen water confirmed that the cell growth inhibitory effect was enhanced by the combined use of the electrolyzed hydrogen water (EHW) and doxorubicin. The results indicate that the autophagy inhibitory activity of the electrolyzed hydrogen water is involved in the enhancement of the effect of doxorubicin.

### (5-2) Verification of Apoptosis Induction

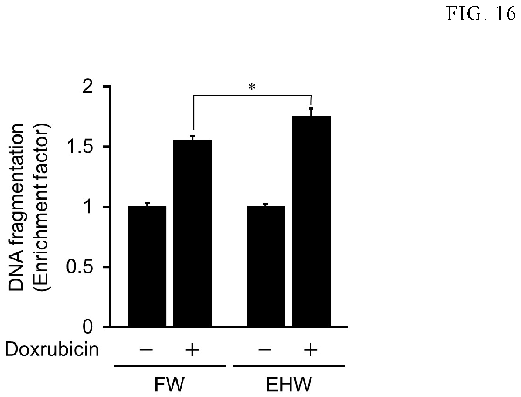

Apoptosis induction was assessed using Cell Death Detection ELISAPLUS (Roche Diagnostics, Basel, Switzerland) according to the manual. Caco-2 cells were seeded into a 12-well plate at 2.0 × 10⁵ cells/well and cultured (precultured) in DMEM under the conditions of 37°C and 5% CO₂ for 24 hours. After the preculture, the medium was exchanged with various DMEMs shown below, and the cells were cultured for 24 hours. The cells were then lysed with a lysis buffer, and the lysate was added to each well of the ELISA plate, incubated with an immunological reagent buffer containing anti-histone biotin and anti-DNA POD for 2 hours, and washed with an incubation buffer. Then, the resultant was reacted with 100 µL of an ABTS substrate buffer, and the absorbance was measured at 405 nm and 490 nm. FIG. 16 shows the results.

In FIG. 16, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which no doxorubicin was added (-) or 2 µM doxorubicin was added (+), and EHW represents DMEM containing electrolyzed hydrogen water with a dissolved hydrogen concentration level of 4 (electrolyzed hydrogen water medium), to which no doxorubicin was added (-) or 2 µM doxorubicin was added (+).

FIG. 16 confirmed that the apoptosis-inducing effect was enhanced by the combined use of doxorubicin and the electrolyzed hydrogen water (EHW).

### (5-3) Analysis of Autophagic Activity

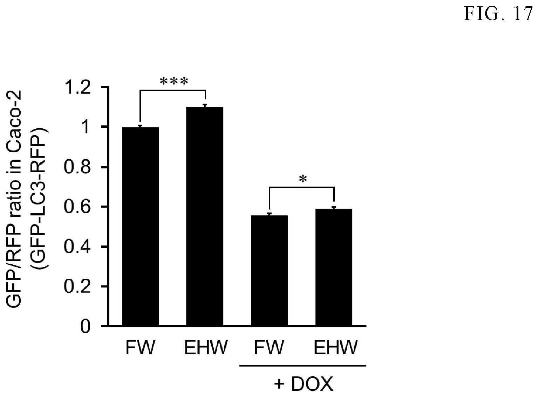

Caco-2 cells expressing the GFP-LC3-RFP probe were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 4 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 17 shows the results.

In FIG. 17, FW represents DMEM containing filtered water (filtered-water medium) diluted with filtered water alone in the section of <Preparation of Medium for Treating Electrolyzed Hydrogen Water (Electrolyzed Hydrogen Water Medium)>, EHW represents DMEM containing electrolyzed hydrogen water with a dissolved hydrogen concentration level of 4 (electrolyzed hydrogen water medium), and + DOX represents the above DMEMs to which 2 µM doxorubicin was added.

FIG. 17 confirmed that the electrolyzed hydrogen water inhibited the autophagy activation of doxorubicin because the combined use of the electrolyzed hydrogen water and the anticancer agent doxorubicin (EHW + DOX) increased the GFP/RFP ratio.

### (5-4) Conclusion

In consideration of the results in the above sections (5-1) to (5-3), the electrolyzed hydrogen water is expected to enhance the anticancer effect by inhibiting the autophagy activating effect of the anticancer agent such as doxorubicin also in the human colon cancer cells (Caco-2 cells), as in the human cervical cancer cells (HeLa cells).

### <6. Verification of Substance Contributing to Enhancing Effect of Electrolyzed Hydrogen Water>

The active molecules having the above effect of the electrolyzed hydrogen water were verified.

### (6-1) Analysis of Autophagic Activity

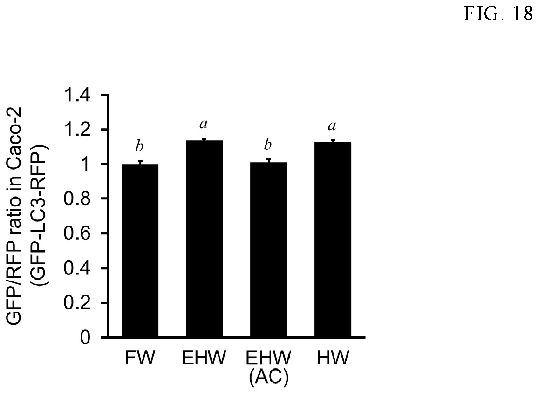

Caco-2 cells expressing the GFP-LC3-RFPΔG probe were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 4 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 18 shows the results.

In FIG. 18, FW represents DMEM containing filtered water (filtered-water medium) diluted with filtered water alone in the section of <Preparation of Medium for Treating Electrolyzed Hydrogen Water (Electrolyzed Hydrogen Water Medium)>, EHW represents DMEM containing electrolyzed hydrogen water with a dissolved hydrogen concentration level of 4 (electrolyzed hydrogen water medium), and EHW (AC) represents autoclaved water-containing DMEM (autoclaved water medium) obtained by diluting the electrolyzed hydrogen water having a dissolved hydrogen concentration level of 4 twice with autoclaved water alone in the section of <Preparation of Medium for Treating Electrolyzed Hydrogen Water (Electrolyzed Hydrogen Water Medium)>, HW represents DMEM (produced hydrogen water medium) containing hydrogen equivalent to the dissolved hydrogen concentration level of 4 obtained by dilution with the produced hydrogen water produced using a kit TRIM SEVEN WATER (manufactured by Trim Japan, Ltd.) alone in the section of <Preparation of Medium for Treating Electrolyzed Hydrogen Water (Electrolyzed Hydrogen Water Medium)>, and Different symbols (a and b) indicate statistically significant differences (p < 0.05). The autoclave treatment was performed twice, for 20 minutes each time, at a high pressure and a high temperature of 121°C.

FIG. 18 confirmed that the electrolyzed hydrogen water (EHW) and the produced hydrogen water (HW) had the same GFP/RFP ratio, and similarly inhibited the autophagy activity. On the other hand, when the electrolyzed hydrogen water was autoclaved twice (EHW (AC)), the GFP/RFP ratio was equivalent to that of the filtered water (FW), indicating that the autophagy inhibitory activity was lost. From the results, the autophagy-inhibiting component in the electrolyzed hydrogen water is assumed to be volatile like molecular hydrogen that is removed by the autoclave treatment.

### (6-2) Verification of Cell Growth Inhibitory Effect

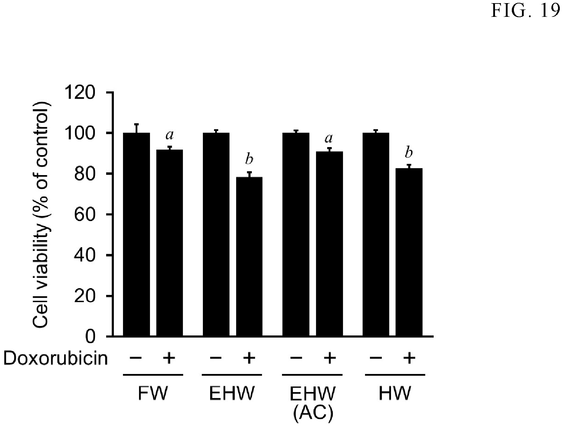

Caco-2 cells were seeded into a 96-well plate at 1.0 × 10⁴ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIG. 19 shows the results.

In FIG. 19, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which no doxorubicin was added (-) or 2 µM doxorubicin was added (+), EHW represents DMEM containing electrolyzed hydrogen water with a dissolved hydrogen concentration level of 4 (electrolyzed hydrogen water medium), to which no doxorubicin was added (-) or 2 µM doxorubicin was added (+), EHW (AC) represents the autoclaved water-containing DMEM (autoclaved water medium), to which no doxorubicin was added (-) or 2 µM doxorubicin was added (+), HW represents DMEM containing hydrogen equivalent to the dissolved hydrogen concentration level of 4 (produced hydrogen water medium), to which no doxorubicin was added (-) or 2 µM doxorubicin was added (+), and Different symbols (a and b) indicate statistically significant differences (p < 0.05).

FIG. 19 confirms that the electrolyzed hydrogen water (EHW) and the produced hydrogen water (HW) equally enhanced the cell growth inhibitory effect of doxorubicin. On the other hand, it is considered that the enhancement effect was lost by the double-autoclaved electrolyzed hydrogen water (EHW (AC)).

### (6-3) Verification of Cell Growth

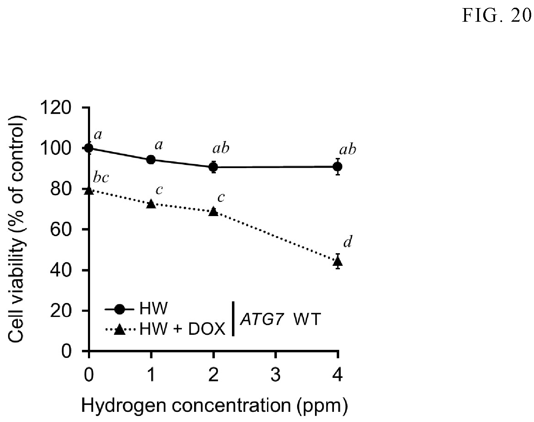

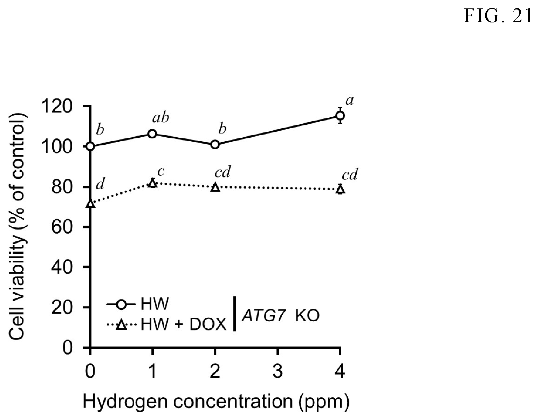

HeLa cells (ATG7 WT) or HeLa cells in which ATG7 (autophagy-related gene) was knocked out (ATG7 KO) were seeded into a 96-well plate at 1.0 × 10⁴ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIGS. 20 and 21 show the results.

In FIGS. 20 and 21, HW represents DMEM (produced hydrogen water medium) containing produced hydrogen water with a dissolved hydrogen concentration of 0 ppm to 4 ppm, and HW + DOX represents the DMEM (produced hydrogen water medium) to which 0.5 µM doxorubicin was added. For example, when a, ab, and b represent Samples 1, 2, and 3, respectively, it is indicated that there is no significant difference between Samples 1 and 2, and between Samples 2 and 3, while Samples 1 and 3 have a significant difference.

FIG. 20 (ATG7 WT) confirmed that the combined use of the produced hydrogen water and the anticancer agent doxorubicin (HW + DOX) inhibited the cell growth as the dissolved hydrogen concentration of the produced hydrogen water increased, indicating enhancement of the cell growth inhibitory effect of doxorubicin. FIG. 21 (ATG7 KO) confirmed that the cell growth inhibitory effect of doxorubicin by the combined use of the produced hydrogen water and the anticancer agent doxorubicin was not exhibited due to the knockout of ATG7 (autophagy-related gene).

### (6-4) Conclusion

In consideration of the results in the above sections (6-1) to (6-3), it was confirmed that dissolved molecular hydrogen was the substance (active molecule) contributing to the enhancement of the anticancer effects (cell growth inhibitory effect and apoptosis induction) of doxorubicin by the electrolyzed hydrogen water, and autophagy inhibition by molecular hydrogen inhibited the cancer cell growth.

### <7. Analysis of Autophagic Activity based on GFP/RFP Ratio Using Other Cells>

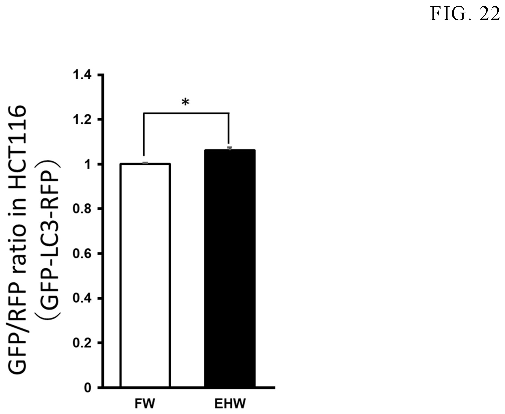

Human colorectal cancer cells expressing the GFP-LC3-RFP probe (hereinafter referred to as "HCT116 cells") were seeded into a 12-well plate at 1.0 × 10⁵ cells and cultured (precultured) in DMEM under the conditions of 37°C and 5% CO₂ for 24 hours. After the preculture, the medium was exchanged with various DMEMs shown below, and the cells were cultured for 4 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 22 shows the results.

In FIG. 22, FW represents DMEM containing filtered water (filtered-water medium) diluted with filtered water alone in the section of <Preparation of Medium for Treating Electrolyzed Hydrogen Water (Electrolyzed Hydrogen Water Medium)>, and EHW represents DMEM containing electrolyzed hydrogen water with a dissolved hydrogen concentration level of 4 (electrolyzed hydrogen water medium).

As shown in FIG. 22, in the human colorectal cancer-derived HCT116 cells, the electrolyzed hydrogen water (EHW) increased the GFP/RFP ratio compared to the filtered water (FW) as in the human cervical cancer-derived HeLa cells (FIG. 4), indicating inhibition of autophagy.

### <8. Analysis of Autophagy Activation and Verification of Anticancer Effect of Other Anticancer Agents>

Whether the other anticancer agents, paclitaxel and fluorouracil, activated autophagy and whether the autophagy activation influenced the anticancer effect were verified.

### (8-1) Analysis of Autophagic Activity of Paclitaxel based on GFP/RFP Ratio Using GFP-LC3-RFP-LC3ΔG Probe

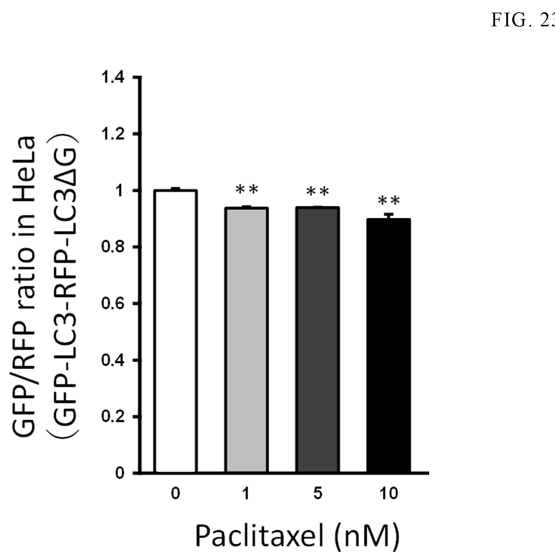

HeLa cells expressing the GFP-LC3-RFP-LC3ΔG probe were seeded into a 12-well plate at 1.0 × 10⁵ cells/well and cultured (precultured) in DMEM under the conditions of 37°C and 5% CO₂ for 24 hours. After the preculture, the medium was exchanged with DMEM supplemented with an anticancer agent containing paclitaxel (Sigma-Aldrich Co., LLC.) at a predetermined concentration (1, 5, or 10 nM), and the cells were cultured for 24 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 23 shows the results.

FIG. 23 shows that the GFP/RFP ratio decreased as the paclitaxel concentration increased, contrary to the electrolyzed hydrogen water (see FIGS. 4 and 22), indicating that paclitaxel has the effect of activating autophagy (autophagy activating effect) similarly to doxorubicin (FIG. 5). From the results, it is predicted that paclitaxel attenuates the anticancer effect by activating autophagy, similarly to doxorubicin.

### (8-2) Verification of Cell Growth Inhibitory Effect

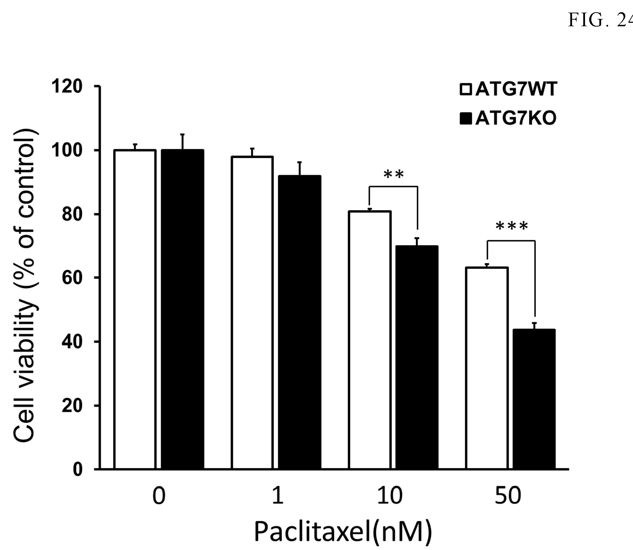

HeLa cells (ATG7 WT) or HeLa cells in which ATG7 (autophagy-related gene) was knocked out (ATG7 KO) were seeded into a 96-well plate at 1.0 × 10⁴ cells/well and cultured (precultured) in DMEM under the conditions of 37°C and 5% CO₂ for 24 hours. After the preculture, the medium was exchanged with DMEM supplemented with an anticancer agent containing paclitaxel at a predetermined concentration (1, 10, or 50 nM), and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIG. 24 shows the results.

The comparison between ATG7 WT and ATG7 KO in FIG. 24 confirmed that the knockout of ATG7 (autophagy-related gene) enhanced the anticancer effect (cell growth inhibitory effect) of paclitaxel in proportion to the concentration.

### (8-3) Analysis of Autophagic Activity of Fluorouracil based on GFP/RFP Ratio Using GFP-LC3-RFP Probe

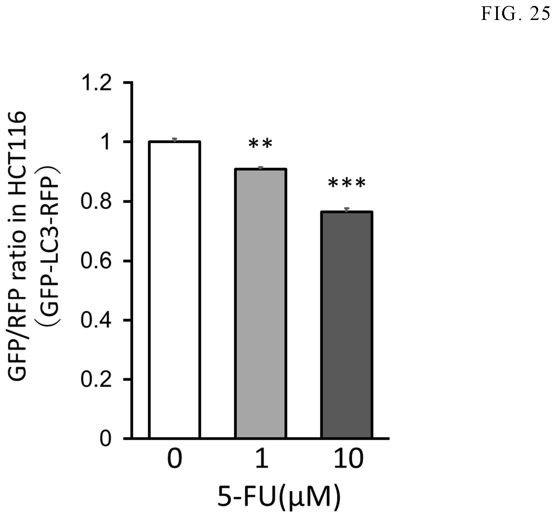

HCT116 cells expressing the GFP-LC3-RFP probe were seeded into a 12-well plate at 1.0 × 10⁵ cells/well and cultured (precultured) in DMEM under the conditions of 37°C and 5% CO₂ for 24 hours. After the preculture, the medium was exchanged with DMEM supplemented with an anticancer agent containing fluorouracil (5-FU, Sigma-Aldrich Co., LLC.) at a predetermined concentration (1 or 10 µM), and the cells were cultured for 24 hours. Then, the cells were detached and suspended in the same manner as described in the section (2-3), and the fluorescence intensities of GFP and RFP were analyzed by flow cytometry. The autophagic activity was quantitatively analyzed by determining the GFP/RFP ratio. FIG. 25 shows the results.

FIG. 25 shows that the GFP/RFP ratio decreased as the fluorouracil concentration increased, contrary to the electrolyzed hydrogen water (see FIG. 22), indicating that fluorouracil has the effect of activating autophagy (autophagy activating effect) similarly to doxorubicin (FIG. 5). From the results, it is predicted that fluorouracil attenuates the anticancer effect by activating autophagy, similarly to doxorubicin.

### <9. Verification of Anticancer Effect Enhancement by Combined Use of Electrolyzed Hydrogen Water and Anticancer Agent Paclitaxel or Fluorouracil>

### (9-1) Verification 1 of Cell Growth Inhibitory Effect

Whether the combined use of electrolyzed hydrogen water and the anticancer agent paclitaxel (anticancer agent kit) enhanced the anticancer effect on the human cervical cancer-derived HeLa cells was verified.

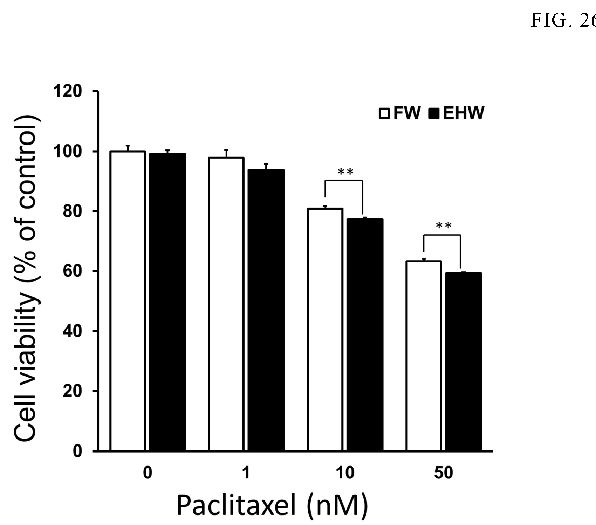

HeLa cells (ATG7 WT) or HeLa cells in which ATG7 (autophagy-related gene) was knocked out (ATG7 KO) were seeded into a 96-well plate at 1.0 × 10⁴ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. The results are shown in FIGS. 26 (ATG7 WT) and 27 (ATG7 KO).

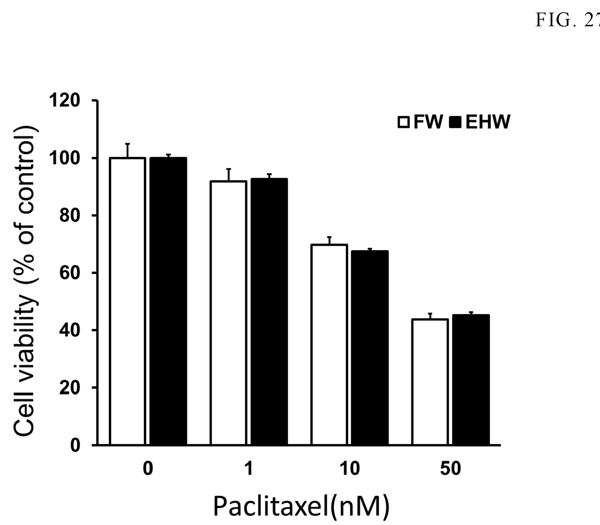

In FIGS. 26 and 27, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which paclitaxel was added at a predetermined concentration (1, 10, or 50 nM), and EHW represents DMEM containing electrolyzed hydrogen water with a dissolved hydrogen concentration level of 4 (electrolyzed hydrogen water medium), to which paclitaxel was added at the predetermined concentration.

FIG. 26 (ATG7 WT) confirmed that the combined use of the electrolyzed hydrogen water and paclitaxel enhanced the cell growth inhibitory effect of paclitaxel. FIG. 27 (ATG7 KO) confirmed that the enhancing effect of the combined use was lost due to the knockout of ATG7 (autophagy-related gene).

### (9-2) Verification 2 of Cell Growth Inhibitory Effect

Whether the combined use of electrolyzed hydrogen water and the anticancer agent fluorouracil (anticancer agent kit) enhanced the anticancer effect on the human colorectal cancer-derived HCT116 cells was verified.

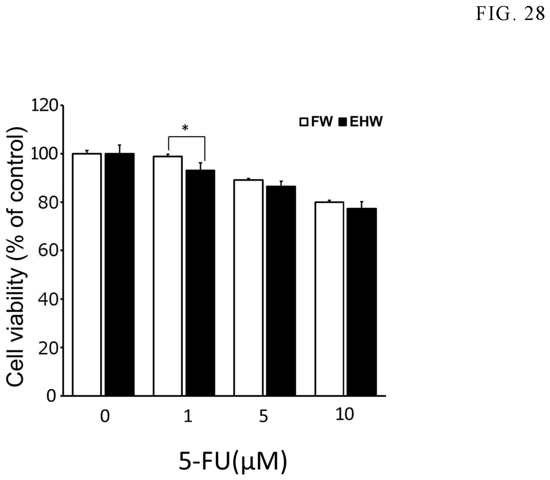

HCT116 cells were seeded into a 12-well plate at 1.0 × 10⁵ cells/well and cultured (precultured) in DMEM under the conditions of 37°C and 5% CO₂ for 24 hours. After the preculture, the medium was exchanged with various DMEMs shown below, and the cells were cultured for 24 hours. Thereafter, 10 µL of Cell Counting Kit-8 (Dojindo, CK04) was added to each well, and the plate was incubated at 37°C for 2 hours, followed by measurement of absorbance at 420 nm. FIG. 28 shows the results.

In FIG. 28, FW represents DMEM containing filtered water (filtered-water medium) diluted with filtered water alone in the section of <Preparation of Medium for Treating Electrolyzed Hydrogen Water (Electrolyzed Hydrogen Water Medium)>, to which fluorouracil was added at a predetermined concentration (1, 5, or 10 µM), and EHW represents DMEM containing electrolyzed hydrogen water with a dissolved hydrogen concentration level of 4 (electrolyzed hydrogen water medium), to which fluorouracil was added at the predetermined concentration.

FIG. 28 confirmed that the combined use of the electrolyzed hydrogen water and fluorouracil enhanced the cell growth inhibitory effect of fluorouracil.

In consideration of the results in the above sections (9-1) and (9-2), the two anticancer agent kits are expected to enhance the anticancer effect by inhibiting the autophagy activating effect of the anticancer agent such as paclitaxel and fluorouracil with the electrolyzed hydrogen water.

### INDUSTRIAL APPLICABILITY

The present invention is applicable as an anticancer agent kit including an anticancer activity enhancing substance and an anticancer agent.

## Claims

1. An anticancer agent kit, comprising:
an anticancer activity enhancer having autophagy inhibitory activity and containing molecular hydrogen as an anticancer activity enhancing substance; and
an anticancer agent, wherein
a concentration of the molecular hydrogen relative to a reference active ingredient concentration (1 µM) of the anticancer agent is 105 ppb or more and 2,160 ppb or less,
targeted cancer cells are at least one selected from the group consisting of human cervical cancer-derived HeLa cells, human colon cancer-derived Caco-2 cells, and human colorectal cancer-derived HCT116 cells, autophagy of which is inhibited by the molecular hydrogen,
the anticancer activity enhancer is electrolyzed hydrogen water,
the anticancer agent contains, as an active ingredient, at least one selected from the group consisting of doxorubicin, bosutinib, ponatinib, cisplatin, vincristine, paclitaxel, dasatinib, bosutinib, nilotinib, and fluorouracil, each of which has an autophagy activating effect, and
the molecular hydrogen enhances an anticancer effect of the anticancer agent by inhibiting the autophagy activating effect of the anticancer agent.

2. The anticancer agent kit of claim 1, excluding an anticancer agent kit that is targeted at colon cancer cells and includes an anticancer agent containing fluorouracil as the active ingredient.

3. An anticancer agent kit, comprising:
an anticancer activity enhancer having autophagy inhibitory activity and containing molecular hydrogen as an anticancer activity enhancing substance; and
an anticancer agent, wherein
a concentration of the molecular hydrogen relative to a reference active ingredient concentration (1 µM) of the anticancer agent is 105 ppb or more and 2,160 ppb or less,
targeted cancer cells are at least one selected from the group consisting of human cervical cancer-derived HeLa cells, human colon cancer-derived Caco-2 cells, and human colorectal cancer-derived HCT116 cells, autophagy of which is inhibited by the molecular hydrogen,
the anticancer activity enhancer is electrolyzed hydrogen water,
the anticancer agent contains, as an active ingredient, at least one selected from the group consisting of doxorubicin, bosutinib, ponatinib, cisplatin, vincristine, paclitaxel, dasatinib, bosutinib, and nilotinib, each of which has an autophagy activating effect, and
the molecular hydrogen enhances an anticancer effect of the anticancer agent by inhibiting the autophagy activating effect of the anticancer agent.

4. The anticancer agent kit of any one of claims 1 to 3, wherein
a concentration of the active ingredient in the anticancer agent is 0.5 µM or more and 10 µM or less.

5. The anticancer agent kit of any one of claims 1 to 3, wherein
the concentration of the active ingredient in the anticancer agent is 0.5 µM or more and 10 µM or less, and
the concentration of the molecular hydrogen is 1,056 ppb or more and 1,080 ppb or less.

6. The anticancer agent kit of any one of claims 1 to 3, wherein
the concentration of the active ingredient in the anticancer agent is 0.5 µM to 2 µM when the human cervical cancer-derived HeLa cells are the targeted cancer cells,
the concentration of the active ingredient in the anticancer agent is 1 µM to 4 µM when the human colon cancer-derived Caco-2 cells are the targeted cancer cells, and
the concentration of the active ingredient in the anticancer agent is 1 µM to 10 µM when the human colorectal cancer-derived HCT116 cells are the targeted cancer cells.
